# DEMANDE DE BREVET EUROPEEN

(11) **EP 1 767 191 A1**
(43) Date de publication de la demande: **28.03.2007**
(21) Numéro de dépôt: 06120065.5
(22) Date de dépôt: 04.09.2006
(51) Int. Cl.: A61K 8/89, A61Q 1/02

(54) **Kit de maquillage comprenant deux compositions cosmétiques**

(30) Priorité: 27.09.2005 FR 0552908
(71) Demandeur: L'Oreal, 75008 Paris (FR)
(72) Inventeur: Themens, Agnès, 92340, Bourg La Reine (FR); Geffroy, Nathalie, 75014 Paris (FR)
(74) Mandataire: Boulard, Denis

(57) **Abrégé**

La présente invention a pour objet un kit de maquillage de la peau, comprenant une première et une seconde compositions cosmétiques, la première composition comprenant au moins un élastomère de silicone et des particules concaves en un matériau siliconé, notamment sous forme de portions de sphères creuses, la seconde composition comprenant un milieu physiologiquement acceptable. Ce kit permet d'obtenir un maquillage uniforme, et/ou masquant les défauts de la peau et/ou évitant le marquage des rides ou ridules.

## Description

La présente invention a pour objet un kit de maquillage de la peau, constitué d'au moins deux compositions destinées à être appliquées l'une sur l'autre, l'une des compositions comprenant un élastomère de silicone et des particules concaves siliconées.

L'invention se rapporte également à un procédé cosmétique de maquillage de la peau, comprenant l'application des compositions sur la peau et à son utilisation pour le maquillage.

Les compositions de maquillage, en particulier de fond de teint, contiennent généralement une phase pulvérulente comprenant des pigments et des charges. Elles sont utilisées pour masquer les imperfections de couleur de la peau, telles que des rougeurs ou les dyschromies. L'application de ces compositions entraîne souvent un marquage des irrégularités de relief de la peau telles que les rides ou les ridules dû à la migration de la phase particulaire à l'intérieur des rides.

Afin de limiter ces problèmes de marquage, il est connu de diminuer la teneur en matière pulvérulente. Dans ce cas, les compositions de fond de teint ne plus masquent plus efficacement les défauts de couleurs de la peau.

Il existe donc un besoin pour des produits de maquillage camouflant de façon efficace les défauts de relief et de couleur de la peau, tout en évitant le marquage des rides ou ridules.

Les inventeurs ont mis en évidence qu'il était possible d'obtenir de tels produits en utilisant un kit de maquillage contenant une première composition comprenant un élastomère de silicone et des particules siliconées concaves et une seconde composition comprenant une matière colorante pulvérulente.

L'application sur la peau de la première composition permet de camoufler les défauts de couleur et de relief de la peau, et évite le marquage des irrégularités de relief de la peau lors de l'application de la seconde composition.

De façon plus précise, l'invention a pour objet un kit de maquillage de la peau, comprenant une première et une seconde compositions cosmétiques, la première composition comprenant au moins un élastomère de silicone et des particules concaves en un matériau siliconé, notamment sous forme de portions de sphères creuses, la seconde composition comprenant un milieu physiologiquement acceptable.

L'invention a également pour objet, selon un second aspect, un procédé cosmétique (non thérapeutique) de maquillage de la peau, comprenant les étapes suivantes :
i) former un premier dépôt sur lesdites matières kératiniques ;
ii) sur tout ou partie du premier dépôt, former un second dépôt,
lesdits premier et second dépôts résultant de l'application d'une première et seconde compositions du kit tel que décrit précédemment.

L'invention a encore pour objet, selon un troisième aspect, une utilisation d'un kit de maquillage tel que décrit précédemment, pour obtenir un maquillage uniforme, et/ou masquant les défauts de la peau et/ou évitant le marquage des rides ou ridules.

Par milieu physiologiquement acceptable, on entend un milieu compatible avec les matières kératiniques, notamment la peau et les lèvres, et en particulier un milieu cosmétique.

### Particules concaves siliconées

Les particules concaves siliconées présentes dans la première composition selon l'invention sont en particulier des particules de portions de sphères creuses constituées d'un matériau siliconé.

La seconde composition du kit selon l'invention peut comprendre des particules concaves siliconées.

Lesdites particules ont un diamètre moyen inférieur ou égal à 10 µm, notamment allant de 0,05 µm à 10µm, préférentiellement allant de 0,1 à 8 µm, plus préférentiellement allant de 0,2 à 7 µm et de préférence encore allant de 0,5 à 5 µm.

Par diamètre moyen, on entend la plus grande dimension de la particule. Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau organosiliconé est un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif peut être un groupe époxy, un groupe (méth)acryloxy, un groupe alkényle, un groupe mercaptoalkyle, aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.

Comme groupe (méth)acryloxy, on peut citer un groupe 3-méthacryloxypropyl, un groupe 3-acryloxypropyl.

Comme groupe alkényle, on peut citer un groupe vinyl, allyl, isopropényl.

Comme groupe mercaptoalkyle, on peut citer un groupe mercaptopropyl, mercaptoéthyl.

Comme groupe aminoalkyle, on peut citer un groupe 3-(2-aminoéthyl)aminopropyl, un groupe 3-aminopropyl, un groupe N,N-diméthylaminopropyl.

Comme groupe halogénoalkyle, on peut citer un groupe 3-chloropropyl, un groupe trifluoropropyl.

Comme groupe glycéroxy, on peut citer un groupe 3-glycéroxypropyl, un groupe 2-glycéroxyéthyl.

Comme groupe uréido, on peut citer un groupe 2-uréidoéthyl.

Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules d'organosilicone peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

Pour les groupements X et Y des composés (III) et (IV) :
Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant un groupement alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toulène sulfonique, l'acide dodécylbenzènesulfonique, l'acide dodécylsulfonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788. Des portions de sphères creuses en forme de fer à cheval sont aussi décrites dans la demande JP-A-2000-191789.

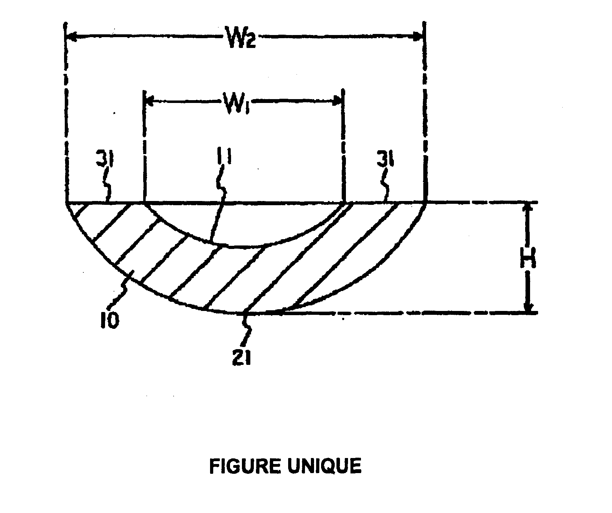

La figure annexée illustre une particule concave de portions de sphères en forme de bol en coupe transversale. La largeur W2 correspond au diamètre des particules.

Comme il ressort de cette figure, ces portions concaves sont formées (en coupe perpendiculaire à un plan de l'ouverture délimitée par la portion de sphère creuse) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne. Les dimensions mentionnées ci-dessus sont obtenues en calculant la moyenne des dimensions de cent particules choisies sur une image obtenue au microscope électronique à balayage.

Comme particules concaves de portions de sphères utilisables selon l'invention, on peut citer :
- les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat)
- les particules constituées de l'organosilicone réticulé TAK-110 (polymère réticulé méthylsilanol/silicate) de la société TAKEMOTO OIL & FAT , en forme de bol , de largeur 0,8 µm, de hauteur 0,4 µm et d'épaisseur 130 nm (particules vendues sous la dénomination NLK-515 par la société Takemoto Oil & Fat)
- les particules constituées de l'organosilicone réticulé TAK-110 (polymère réticulé méthylsilanol/silicate) de la société TAKEMOTO OIL & FAT , en forme de bol , de largeur 7 µm, de hauteur 3,5 µm et d'épaisseur 200 nm (particules vendues sous la dénomination NLK-51 0 par la société Takemoto Oil & Fat)

Ces particules ont pour nom CTFA : methyle silanol/silicate crosspolymer.

Avantageusement, les particules concaves siliconées ont un diamètre moyen inférieur ou égal à 5 µm, notamment allant de 0,1 µm à 5 µm, préférentiellement allant de 0,2 à 5 µm, plus préférentiellement allant de 0,5 à 4 µm, et de préférence encore allant de 0,5 à 3 µm.

Ces particules de diamètre moyen inférieur ou égal à 5 µm permettent d'obtenir un épaississement de la phase grasse plus important que celui obtenu avec des particules de diamètre moyen supérieur à 5 µm. Ces particules permettent d'optimiser les propriétés de glissant, d'étalement, et de confort de la composition selon l'invention.

Les particules concaves siliconées peuvent être présentes dans la première et/ou seconde composition du kit selon l'invention en une teneur allant de 0,1 % à 15 % en poids, par rapport au poids total de la composition, de préférence allant de 0,5 % à 10 % en poids, et préférentiellement allant de 1 % à 8 % en poids.

Selon un mode préféré de réalisation, le rapport massique de la teneur en particules concaves siliconées sur la teneur de l'élastomère de silicone va de 0,1 à 100, de préférence de 0,2 à 50, de façon encore plus préférentielle de 0,5 à 10.

### Elastomères de silicone

La première composition du kit selon l'invention comprend également un élastomère de silicone.

Selon un mode particulier de réalisation, la seconde composition du kit selon l'invention peut comprendre également un élastomère de silicone.

L'élastomère de silicone peut être non émulsionnant ou émulsionnant.

Selon un mode préféré de réalisation, la première composition du kit selon l'invention peut comprendre un élastomère de silicone non émulsionnant.

Le terme élastomères de silicone "non émulsionnant" définit des élastomères organopolysiloxane ne contenant pas de chaîne hydrophile telle que motifs polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.
Les organopolysiloxanes (A2) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxaneméthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).
Avantageusement, la somme du nombre de groupements éthyléniques par molécule du composé (A2) et le nombre d'atomes d'hydrogène liés au silicum par molécule du composé (B2) est d'au moins 4.
Le composé (B2) peut être sous toute structure moléculaire, notamment de structure chaîne linéaire, chaîne ramifiée, structure cyclique.
Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

L'élastomère de silicone non émulsionnant est généralement mélangé avec au moins une huile hydrocarbonée et/ou une huile siliconée pour former un gel. Dans ces gels, l'élastomère non émulsionnant est sous forme de particules non-sphériques.

Des élastomères non émulsionnants sont notamment décrits dans les brevets US4970252, US4987169, US 5412004, US5654362, US5760116, dans la demande JP-A-61-194009.

Comme élastomères non émulsionnants, on peut utiliser ceux vendus sous les dénominations "KSG-6", "KSG-15", "KSG-16", "KSG-18", "KSG-41", "KSG-42", "KSG-43", "KSG-44", "USG-105", "USG-106" par la société Shin Etsu, "DC 9040", "DC9041", "DC 9509", "DC9505", "DC 9506" par la société Dow Corning, "GRANSIL" par la société Grant Industries, "SFE 839" par la société General Electric.

Les particules d'organopolysiloxane réticulés élastomères peuvent également se présenter sous forme de poudre, notamment sous forme de poudre sphérique.

Les particules d'élastomère de silicone peuvent se présenter également sous forme de poudre d'organopolysiloxane réticulé élastomère enrobée de résine de silicone, notamment de résine silsesquioxane, comme décrit par exemple dans le brevet US5538793. De tels élastomère sont vendus sous les dénomination "KSP-100", "KSP-1 01 ", "KSP-102", "KSP-1 03", KSP-104", "KSP-1 05" par la société Shin Etsu.

D'autres élastomère de silicone sous forme de poudres peuvent être des poudres de silicone hybride fonctionnalisé par des groupes fluoroalkyle, notamment vendues sous la dénomination "KSP-200" par la société Shin Etsu ; des poudres de silicones hybride fonctionnalisé par des groupes phényl, notamment vendues sous la dénomination "KSP-300" par la société Shin Etsu.

L'élastomère de silicone non émulsionnant peut être présent dans la première et/ou seconde composition du kit selon l'invention en une teneur allant de 0,1 % à 10 % en poids, par rapport au poids total de la composition, de préférence allant de 0,5 % à 7% en poids, et plus préférentiellement allant de 1 % à 5 % en poids.

La première et/ou seconde composition peut comprendre un élastomère de silicone émulsionnant.

Par élastomère de silicone émulsionnant on entend un élastomère de silicone comprenant au moins une chaîne hydrophile.

L'élastomère de silicone émulsionnant peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est véhiculé sous forme de gel dans au moins une huile hydrocarbonée et/ou une huile siliconée. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.
Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-240","KSG-310", "KSG-320", "KSG-330", "KSG-340", "X-226146" par la société Shin Etsu, "DC9010", "DC9011" par la société Dow Corning.

L'élastomère de silicone émulsionnant peut être également choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁ -O-[Gly]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet WO2004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

L'élastomère de silicone (incluant les élastomères émulsionnants et les non émulsionnants) peut être présent dans la première et/ou seconde composition du kit selon l'invention en une teneur allant de 0, 1 % à 10 % en poids, par rapport au poids total de la composition, de préférence allant de 0,5 % à 7 % en poids, et plus préférentiellement allant de 1 % à 5 % en poids.

### Les huiles

La première et/ou la seconde composition du kit selon l'invention peut comprendre au moins une huile.

L'huile peut être choisie parmi les huiles volatiles, les huiles non volatiles, et leurs mélanges.

La première et/ou seconde composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le 3-butyl 1,1,1,3,5,5,5-heptaméthyl trisiloxane, le 3-propyl 1,1,1,3,5,5,5-heptaméthyl trisiloxane, le 3-éthyl 1,1,1,3,5,5,5-heptaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Les huiles volatiles sont présentes dans la première et/ou seconde composition du kit selon l'invention en une teneur allant de 6 à 45 % en poids par rapport au poids total de la composition, de préférence allant de 6 à 40 % en poids et plus préférentiellement allant de 6 à 35 % en poids.

La première et/ou seconde composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges. Comme huile hydrocarbonée non volatile préférée, on utilise des esters de formule R₁COOR₂dans laquelle R₁ représente le reste d'un acide gras ramifié comportant de 6 à 10 atomes de carbone et R₂ représente une chaîne hydrocarbonée (ou un mélange de chaînes hydrocarbonées) contenant de 10 à 18 atomes de carbone, de préférence de 12 à 16 atomes de carbone.

L'huile (ou le mélange d'huiles) de la première et/ou seconde composition selon l'invention peut être présente en une teneur allant de 1 % à 80 % en poids, par rapport au poids total de la composition, de préférence allant de 5 % à 60 % en poids, de préférence allant de 5 % à 50 % en poids.

Selon un mode préféré de réalisation, la première composition du kit selon l'invention comprend au moins une huile volatile.

Selon un mode particulier de réalisation, la seconde composition selon l'invention comprend au moins une huile de silicone volatile et une huile non volatile d'ester d'alkyle en C₁₀-C₁₈, en particulier un ester d'alkyle en C₁₂-C₁₅.

Selon un mode préféré de réalisation, l'ester d'alkyle en C₁₀-C₁₈ (en particulier en C₁₂-C₁₅) peut être présent dans la seconde composition en une teneur allant 5 à 20% en poids, par rapport au poids total de la composition.

### Phase aqueuse

Les première et/ou seconde compositions du kit selon l'invention peuvent comprendre une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse de la première et de la seconde compositions peut comprendre au moins un solvant organique miscible à l'eau (à température ambiante - 25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

Selon un mode préféré de réalisation, la phase aqueuse de la première et/ou de la seconde compositions peut comprendre un polyol hydratant choisi parmi les polyols de préférence ayant de 2 à 10 atomes de carbone, et préférentiellement ayant de 2 à 6 atomes de carbone. En particulier, le solvant organique miscible à l'eau est choisi parmi le glycérol, le propylène glycol, le butylène glycol, et leurs mélanges.

La première et/ou la seconde composition du kit selon l'invention peut comprendre au moins un polyol hydratant en une teneur inférieure ou égale à 15% en poids, par rapport au poids total de chaque composition, de préférence allant de 4 à 15% en poids, plus préférentiellement allant de 5 à 12% en poids, et plus préférentiellement allant de 8 à 12% en poids.

Selon un mode encore préféré de réalisation, chacune des première et seconde composition du kit selon l'invention comprend au moins un polyol hydratant.

Selon un mode particulier de réalisation, la somme des teneurs en polyols hydratants des première et seconde compositions, exprimées en poids, par rapport au poids total de chaque composition, est supérieure ou égale à 8, en particulier va de 8 à 30, de préférence de 10 à 25, et plus préférentiellement de 15 à 20.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la première et/ou la seconde composition du kit selon l'invention en une teneur allant de 5 à 80 % en poids par rapport au poids total de la composition et de préférence allant de 10 à 70 % en poids.

De préférence, l'eau peut être présente dans la première et/ou la seconde composition selon l'invention en une teneur allant de 10 à 75 % en poids par rapport au poids total de la composition, de préférence allant de 15 à 65 % en poids.

### LES MATIERES COLORANTES

La seconde composition du kit selon l'invention peut comprendre une matière colorante pulvérulente.

Selon un mode particulier de réalisation, la première composition peut comprendre une matière colorante pulvérulente.

On entend par matière colorante au sens de la présente invention, un composé susceptible de produire un effet optique coloré lorsqu'il est formulé en quantité suffisante dans un milieu cosmétique approprié.

La matière colorante pulvérulente peut être notamment choisie parmi les pigments, les nacres, et leurs mélanges.

Par « pigments », il faut comprendre des particules blanches ou colorées, minérales
ou organiques, insolubles dans la phase organique liquide, destinées à colorer et/ou opacifier la composition.
Par « nacres », il faut comprendre des particules irisées, notamment produites par certains mollusques dans leur coquille ou bien synthétisées, insolubles dans le milieu de la composition.

Selon un mode de réalisation de l'invention, la matière colorante comprend au moins un pigment.

Les pigments peuvent être choisis parmi les pigments minéraux, les pigments organiques, et les pigments composites (c'est-à-dire des pigments à base de matériaux minéraux et/ou organiques).

Par pigments, il faut comprendre des particules de toute forme, dotées d'un effet optique, minérales ou de synthèse, insolubles dans le milieu de la composition quelle que soit la température à laquelle la composition est fabriquée.

Les pigments peuvent être choisis parmi les pigments monochromes, les laques, les nacres, les pigments à effet optiques, comme les pigments réfléchissants et les pigments goniochromatiques.

Les pigments minéraux peuvent être choisis parmi les pigments d'oxyde métallique, le mica recouvert de dioxyde de titane, le mica recouvert d'oxychlorure de bismuth, le mica titane recouvert d'oxyde de fer, le mica-titane recouvert de bleu ferrique, le mica titane recouvert d'oxyde de chrome, les oxydes de fer, le dioxyde de titane, les oxydes de zinc, l'oxyde de cérium, l'oxyde de zirconium, l'oxyde de chrome ; le violet de manganèse, le bleu de prusse, le bleu outremer, le bleu ferrique, l'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.

Les pigments organiques peuvent être par exemple :
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique ;
- les pigments mélaniques.

Parmi les pigments organiques, on peut citer les D&C Blue n°4, D&C Brown n° 1, D&C Green n°5, D&C Green n°6, D&C Orange n°4 , D&C Orange n°5 , D&C Orange n °10 , D&C Orange n°11 , D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, D&C Violet n°2, D&C Yellow n°7, D&C Yellow n°8, D&C Yellow n°10, D&C Yellow n°11, FD&C Blue n° 1, FD&C Green n°3, FD&C Red n °40 , FD&C Yellow n °5, FD&C Yellow n °6.

Selon un mode préféré de réalisation, les pigments présents dans les premières et/ou seconde compositions selon l'invention sont des pigments enrobés hydrophobes.

Par pigments enrobés hydrophobes, on entend des pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

Les pigments destinés à être enrobés peuvent être des pigments minéraux ou organiques décrits ci-dessus.

On utilise de préférence des pigments d'oxydes de fer ou de dioxyde de titane.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges. Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.
La première et/ou seconde composition du kit selon l'invention peut comprendre des nacres.

Les nacres peuvent être choisies parmi les pigments nacrés blancs tels que le mica recouvert de titane ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les pigments et/ou les nacres peuvent être présents dans la première composition en une teneur allant de 0,01 à 6 % en poids, par rapport au poids total de la première composition, et de préférence allant de 0,5 à 5 % en poids, et préférentiellement allant de 1 à 3 % en poids.

Selon un mode préféré de réalisation, la matière colorante pulvérulente de la première composition du kit selon l'invention comprend au moins une nacre.

Selon un mode préféré de réalisation, la première composition du kit selon la présente invention comprend moins de 5% en poids, par rapport au poids total de la composition, de pigments, notamment de 0,1 à 5 % en poids, mieux de 0,1 à 3% en poids de pigments.

Selon un mode préféré de réalisation, la première composition du kit selon la présente invention ne contient pas de pigment.

Les pigments et/ou les nacres peuvent être présents dans la seconde composition en une teneur allant de 0,01 à 20 % en poids, par rapport au poids total de la première composition, et de préférence allant de 0,5 à 18 % en poids, et préférentiellement allant de 3 à 15 % en poids.

Les matières colorantes peuvent également comprendre des colorants hydro ou liposolubles.

Les matières colorantes pulvérulentes peuvent être présentes dans la première et/ou seconde composition en une teneur allant de 0,01 % à 25 % en poids, par rapport au poids total de la composition, de préférence allant de 0,5 % à 20 % en poids, et préférentiellement allant de 1 à 18 % en poids.

### Charges

La première et/ou seconde composition du kit selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®), les poudres de poly-β-alanine, les poudres de polyéthylène, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les particules de polyméthacrylate de méthyle, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Les charges peuvent être présentes dans la première et/ou seconde composition du kit en une teneur allant de 0,5 % à 25 % en poids, par rapport au poids total de la composition, de préférence allant de 1 % à 20 % en poids, et préférentiellement allant de 2 % à 15 % en poids.

Selon un mode préféré de réalisation, la seconde composition du kit selon l'invention comprend moins de 5% en poids, par rapport au poids total de la composition, de charges absorbantes, notamment de 0,1 à 5% en poids, et mieux de 0,1 à 3% en poids. Selon un mode plus préféré de réalisation, la seconde composition du kit selon l'invention est exempte de charges absorbantes.

On appelle charges absorbantes ou « pompes à sébum » au sens de la présente demande un composé apte à absorber et/ou adsorber le sébum.
Généralement ce type de composé se présente sous la forme d'une poudre ayant une prise de sébum.
De manière avantageuse, la prise de sébum de ces composés est supérieure ou égale à 1 ml/g, et peut notamment varier de 1 ml/g à 20 ml/g, en particulier de 1 ml/g à 15 ml/g. Elle peut notamment être supérieure ou égale à 1,5 ml/g, et en particulier varier de 1,5 ml/g à 20 ml/g, voire varier de 1,5 ml/g à 15 ml/g.
De telles charges sont notamment décrites dans la demande de brevet EP-A-1559393 dont le contenu est introduit par référence dans la présente demande.

Elles peuvent notamment être choisies parmi : la silice, les poudres de polyamide (nylon), les poudres de polymères acryliques, notamment de polyméthacrylate de méthyle, de polyméthacrylate de méthyle/diméthacrylate d'éthylène glycol, de polyméthacrylate d'allyle/diméthacrylate 5 acrylate d'éthylène glycol de copolymère diméthacrylate d'éthylène glycol/méthacrylate de lauryle et les poudres de polyéthylène, notamment de polyéthylène/acide acrylique.

A titre représentatif et non limitatif des charges absorbantes selon l'invention, on peut tout particulièrement citer :
- les poudres de silice, comme par exemple les microsphères de silice poreuses vendues sous la dénomination "SILICA BEADS SB-700" commercialisées par la société MYOSHI, les "SUNSPHERE H51 ", "SUNSPHERE H33" , "SUNSPHERE H53" commercialisées par la société ASAHI GLASS ; et les microsphères de silice amorphe enrobées de polydiméthylsiloxane 5 ne vendues sous la dénomination "SA SUNSPHERE H-33" et "SA SUNSPHERE H-53" par la société ASAHI GLASS,
- les poudres de polyamides (nylon), comme par exemple l'"ORGASOL 4000" ou l"'ORGASOL 2002 EXTRA D NAT COS" commercialisés par la société ATOCHEM,
- les poudres de polymères acryliques, notamment de polyméthacrylate de méthyle, comme par exemple le "COVABEAD LH85" commercialisé par la société WACKHERR ; de polyméthacrylate de méthyle/diméthacrylate d'éthylène glycol, comme par exemple le "DOW CORNING 5640 MICROSPONGE SKIN OIL ADSORBER" commercialisé par la société DOW CORNING, ou le "GANZPEARL GMP-0820" commercialisé par la société GANZ CHEMICAL ; de polyméthacrylate d'allyle/diméthacrylate d'éthylène glycol, comme par exemple le "POLY-PORE L200" ou le "POLY-PORE E200" commercialisés par la société AMCOL ; de copolymère diméthacrylate d'éthylène glycol/méthacrylate de lauryle, comme par exemple le "POLYTRAP 6603" commercialisé de la société DOW CORNING, et
- les poudres de polyéthylène, notamment de polyéthylène/acide acrylique vendues sous le nom commercial Flobeads® par la société SUMITOMO.

### Viscosité de la seconde composition du kit

Avantageusement, la seconde composition selon l'invention présente une viscosité, mesurée à 25 °C, à un gradient de cisaillement de 200 s⁻¹, allant de 0,1 à 0,8 Pa.s (1 à 8 poises), et de préférence allant de 0,2 à 0,7 Pa.s (2 à 7 poises). Une telle viscosité permet une application bien glissante de la composition sur la peau. La viscosité est mesurée avec un rhéomètre à contrainte imposée RHEOSTRESS RS 600 de la société THERMO utilisant un cône plan sablé de 60mm, présentant un angle de 2° avec un entrefer de 0,105mm.

### Galénique

La première composition du kit selon l'invention se présente sous forme d'une émulsion eau-dans-huile ou huile-dans-eau, d'une composition anhydre (poudre, stick, gel huileux) et de préférence eau-dans-huile.

La seconde composition du kit peut se présenter sous forme d'une émulsion (huile-dans-eau ou eau-dans-huile), d'un gel aqueux ou d'un anhydre et de préférence eau-dans-huile.

Selon un mode particulier de réalisation, les phases continues des première et seconde compositions sont de même nature.

Selon un mode plus particulier de réalisation, les phases continues première et seconde compositions sont huileuses.

Selon un mode préféré de réalisation, la seconde composition se présente sous forme d'une émulsion eau-dans-huile.

Selon un mode préféré de réalisation, le kit selon l'invention peut comprendre :
i) Une première composition sous forme d'émulsion eau-dans-huile comprenant :
   - 0,1 à 10% en poids, par rapport au poids total de la composition, d'au moins un élastomère de silicone,
   - 0, 1 % à 15 % en poids, par rapport au poids total de la composition, de particules concaves en un matériau siliconé, notamment sous forme de portions de sphères creuses
   - au moins une polyol hydratant, et
   - comprenant moins de 5% en poids, par rapport au poids total de la composition, de pigments ; et
ii) Une seconde composition sous forme d'émulsion eau-dans-huile comprenant :
   - 0,01 à 20 % en poids, par rapport au poids total de la composition, de pigments et/ou nacres,
   - au moins un polyol hydratant, et
   - comprenant moins de 5% en poids, par rapport au poids total de la composition, de charges absorbantes.

### Additifs

La première et/ou seconde composition selon l'invention peut comprendre au moins un autre ingrédient cosmétique usuel pouvant être choisi notamment parmi les agents gélifiants et/ou épaississants hydrophiles ou lipophiles, les antioxydants, les parfums, les conservateurs, les neutralisants, les filtres solaires, les vitamines, les composés auto-bronzants, les actifs antirides, les émollients, les cires, les actifs hydrophiles ou lipophiles, les agents anti-pollution ou anti-radicaux libres, les sequestrants, les agents filmogènes, les tensioactifs, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

L'invention est illustrée plus en détail dans les exemples ci-après. Les teneurs sont exprimées en pourcentages massiques.

### Exemples de kits de maquillage :

### Exemples 1 et 2 : premières compositions du kit selon l'invention

| | **Ex. 1** | **Ex. 2** |
|---|---|---|
| ***Phase huileuse*** | | |
| Gel d'élastomère de silicone - copolymère diméthicone/vinyl diméthicone (25%) dans polydiméthylsiloxane 6cst vendu sous la dénomination KSG-16 par la société SHIN ETSU | 10 | 12,6 |
| Cyclopentasiloxane | 18,25 | 18,05 |
| Polydiméthylsiloxane 10 cSt | 0 | 2 |
| Isododécane | 4,6 | 0 |
| Mélange de cétyl diméthycone copolyol, d'isostéarate de polyglycéryle-4 et de laurate d'hexyle (30/40/30) vendu sous la dénomination Abil WE 09 par la société GOLDSCHMIDT | 9 | 9 |
| Particules concaves siliconées vendues sous la dénomination NLK-506 par la société TAKEMOTO OILS & FAT | 4 | 4 |
| Micro sphères creuses de poly méthacrylate de méthyle vendues sous le nom COVABEAD LH85 par la société LCW | 5 | 5 |
| Nacre | 0 | 0,2 |
| | | |

| ***Phase aqueuse*** | | |
|---|---|---|
| Conservateur | qs | qs |
| Propylène glycol | 5 | 5 |
| Stéarate d'éthylène glycol acétylé | 0,7 | 0,7 |
| Sulfate de magnésium | 0,7 | 0,7 |
| Eau | qsp 100% | qsp 100% |

### Mode opératoire :

On fait fondre le gel d'élastomère de silicone, le mélange de cétyl diméthycone copolyol, d'isostéarate de polyglycéryle-4 et de laurate d'hexyle et le stéarate d'éthylène glycol acéthylé. On y ajoute la cyclopentasiloxane, l'isododécane et la polydiméthylsiloxane. Puis on disperse les microsphères de PMMA, les particules concaves siliconées et la nacre dans le mélange. Le mélange obtenu constitue alors la phase grasse de l'émulsion.
On mélange séparément les autres constituants (phase aqueuse), puis on verse ce mélange dans la phase grasse suivant un procédé classique d'émulsification au Moritz.

### Exemple 3 à 6 : secondes compositions du kit selon l'invention

| | Exemple 3 | Exemple 4 | Exemple 5 | Exemple 6 |
|---|---|---|---|---|
| ***Phase Huileuse*** | | | | |
| Céthyl diméthicone copolyol vendu sous la denomination Abil EM 90 par la société GOLDSCHMIDT | 0,80 | 0,80 | 0,80 | 0,80 |
| Isostéarate de polyglycéryle-4 vendu sous la dénomination Isolan GI 34 par la société GOLDSCHMIDT | 0,60 | 0,60 | 0,60 | 0,6 |
| Laurate d'hexyle | 0,60 | | | |
| Polydiméthylsiloxane oxyéthyléné vendu sous la denomination KF-6017 par la société Shin Etsu | 5,0 | 5,0 | 5,0 | 5,0 |
| Isoeicosane | 4,50 | | | |
| Dimethicone (5 cSt) | 2,40 | | | |
| Neopentanoate d'isostearyle | 0,50 | | | |
| Isononanoate d'isononyle | | | 19,20 | 10,00 |
| Ether dicaprylique | | 19,20 | | |
| Cyclohexasiloxane | 5,90 | | | |
| Cyclopentane | 10,40 | 18,0 | 18,0 | 18,0 |
| Ethyle 2-hexanoate d'alkyle en C12-C15 vendu sous la dénomination Hetester par la société Heterene Chemical | | | | 9,20 |
| Hectorite modifiée vendue sous la dénomination Bentone 38 VCG par la société Elementis | 1,90 | 1,90 | 1,90 | 1,90 |
| Isododecane | 13,40 | | | |
| vitamine E | 0,10 | 0,10 | 0,10 | 0,10 |
| Pigments enrobés | 11,0 | 11,0 | 11,0 | 11,00 |
| Microbilles de résine polymethylsilsesquioxane vendues sous la dénomination TOSPEARL 145 A par la société GE Toshiba Silicones | 4,0 | | | |
| Micro sphères creuses de poly méthacrylate de méthyle vendues sous le nom COVABEAD LH85 par la société LCW | | 2,0 | 2,0 | 2,0 |

| ***Phase aqueuse*** | | | | |
|---|---|---|---|---|
| Butylène Glycol | 3,0 | 3,0 | 3,0 | 3,0 |
| Glycérine | 7,0 | 7,0 | 7,0 | 7,0 |
| Chlorure de sodium | 0,70 | 0,70 | 0,70 | 0,70 |
| D-Panthenol | 0,10 | 0,10 | 0,10 | 0,10 |
| Conservateur | qs | qs | qs | qs |
| Eau | qsp 100% | qsp 100% | qsp 100% | qsp 100% |

### Mode opératoire :

### Préparation d'une pâte pigmentaire :

On prépare un broyat de pigments dans une partie de cyclopentasiloxane.

### Préparation de la phase grasse :

On mélange au Moritz les phases A1 et A2 à température ambiante.
On ajoute l'hectorite dans le mélange A1+A2 et on maintient l'agitation jusqu'à obtenir un mélange homogène. Puis, on ajoute le broyat pigmentaire et la vitamine E et les charges sous agitation au Moritz pendant 10mn jusqu'a dispersion homogène des microbilles.

### Préparation de la phase aqueuse :

On mélange les constituants de B1 à température ambiante

### Préparation de l'émulsion :

Sous agitation au Moritz et à température ambiante, on verse la phase aqueuse dans la phase grasse. On maintient l'agitation jusqu'à ce que la phase aqueuse soit bien dispersée dans la phase grasse.

On applique sur la peau l'une ou l'autre des compositions des exemples 1 ou 2.
L'application sur la peau de cette première composition permet un bon camouflage des défauts de la peau.
Puis on applique sur le premier dépôt l'une des compositions des exemples 3, 4 ou 5 (seconde composition du kit).
Le maquillage obtenu est uniforme, masque les défauts de la peau et évite le marquage des rides ou ridules.

## Revendications

1. kit de maquillage de la peau, comprenant une première et une seconde compositions cosmétiques, la première composition comprenant au moins un élastomère de silicone et des particules concaves en un matériau siliconé, notamment sous forme de portions de sphères creuses, la seconde composition comprenant un milieu physiologiquement acceptable.

2. Kit selon la revendication précédente, **caractérisé par le fait que** lesdites particules concaves siliconées ont un diamètre moyen inférieur ou égal à 10 µm.

3. Kit selon l'une quelconque des revendications précédentes, **caractérisé par le fait que** lesdites particules concaves siliconées ont un diamètre moyen allant de 0,05 µm à 10µm, préférentiellement allant de 0,1 à 8 µm, plus préférentiellement allant de 0,2 à 7 µm et de préférence encore allant de 0,5 à 5 µm.

4. Kit selon l'une quelconque des revendications précédentes, **caractérisé par le fait que** les particules concaves siliconées sont sous forme de portions de sphères creuses ayant une section transversale en forme de fer à cheval ou d'arceau.

5. Kit selon l'une quelconque des revendications précédentes, **caractérisé par le fait que** le matériau siliconé est un polysiloxane réticulé de structure tridimensionnelle comprenant, ou constitué de, des motifs de formule (l) : SiO₂ et de formule (II) : R¹SiO_{1,5}
dans lesquelles R¹ désigne un groupe organique ayant un atome de carbone directement relié à l'atome de silicium.

6. kit selon la revendication précédente, **caractérisé par le fait que** le groupe organique est un groupe organique réactif, un groupe organique non réactif, et de préférence un groupe organique non réactif.

7. Kit selon la revendication précédente, **caractérisé par le fait que** le groupe organique non réactif peut être un groupe alkyle en C₁-C₄, ou un groupe phényle.

8. Kit selon la revendication 6 ou 7, **caractérisé par le fait que** le groupe organique non réactif est un groupe méthyle.

9. Kit selon la revendication 6, **caractérisé par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

10. Kit selon la revendication 6 ou 9, **caractérisé par le fait que** le groupe organique réactif est choisi parmi un groupe époxy, un groupe (méth)acryloxy, un groupe alkényle, un groupe mercaptoalkyle, aminoalkyle.

11. Kit selon la revendication 5, **caractérisé par le fait que** R¹ désigne un groupe méthyle.

12. Kit selon l'une quelconque des revendications 5 à 11, **caractérisé par le fait que** le matériau siliconé comprend les motifs (I) et (II) selon un rapport molaire motif (I) / motif (II) allant de 30/70 à 50/50, de préférence allant de 35/65 à 45/55.

13. Kit selon l'une quelconque des revendications précédentes, **caractérisé par** le fait les particules concaves en matériau siliconé sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

14. Kit selon la revendication précédente, **caractérisé par le fait que** dans l'étape (a), le rapport molaire du composé (III) au composé (IV) va de 30/70 à 50/50, de préférence va de 35/65 à 45/45, et préférentiellement est de 40/60.

15. kit selon la revendication 13 ou 14, **caractérisé par le fait que** le rapport en poids de l'eau au total des composés (III) et (IV) va de 10/90 à 70/30 dans l'étape (a).

16. Kit selon la revendication 13, **caractérisé par le fait que** le groupe organique est un groupe organique réactif, un groupe organique non réactif, et de préférence un groupe organique réactif.

17. Kit selon la revendication précédente, **caractérisé par le fait que** le groupe organique non réactif peut être un groupe alkyle en C₁-C₄, ou un groupe phényle.

18. Kit selon la revendication 16, **caractérisé par le fait que** le groupe organique non réactif est un groupe méthyle.

19. Kit selon la revendication 16, **caractérisé par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

20. Kit selon la revendication 16 ou 19, **caractérisé par le fait que** le groupe organique réactif est choisi parmi un groupe époxy, un groupe (méth)acryloxy, un groupe alkényle, un groupe mercaptoalkyle, aminoalkyle.

21. Kit selon la revendication 13, **caractérisé par le fait que** R¹ désigne un groupe méthyle.

22. Kit selon l'une quelconque des revendications précédentes, **caractérisé par le fait que** les particules concaves sont formées (en coupe) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

23. Kit selon l'une quelconque des revendications précédentes, **caractérisé par le fait que** les particules concaves siliconées sont présentes dans la première composition en une teneur allant de 0, 1 % à 15 % en poids, par rapport au poids total de la première composition, de préférence allant de 0,5 % à 10 % en poids, et plus préférentiellement allant de 1 à 8% en poids.

24. Kit selon l'une quelconque des revendications précédentes, **caractérisé en ce que** l'élastomère de silicone est un élastomère de silicone non émulsionnant.

25. Kit selon la revendication précédente, **caractérisé en ce que** l'élastomère de silicone non émulsionnant est présent dans la première composition en une teneur allant de 0,1 à 10% en poids, par rapport au poids total de la composition, de préférence allant de 0,5 à 7 % en poids, et plus préférentiellement allant de 1 à 5% en poids.

26. Kit selon l'une quelconque des revendications précédentes, **caractérisé en ce que** la première et/ou la seconde composition comprend au moins une huile.

27. kit selon la revendication précédente, **caractérisé en ce que** la première et/ou la seconde composition comprend une huile volatile siliconée.

28. Kit selon la revendication 26, **caractérisé en ce que** la première et/ou la seconde composition comprend une huile ester de formule R₁COOR₂dans laquelle R₁ représente le reste d'un acide gras ramifié comportant de 6 à 10 atomes de carbone et R₂ représente une chaîne hydrocarbonée, ou un mélange de chaînes hydrocarbonées, contenant de 10 à 18 atomes de carbone, de préférence de 12 à 16 atomes de carbone.

29. Kit selon les revendications 26 à 28, **caractérisé en ce que** l'huile (ou le mélange d'huiles) est présente en une teneur allant de 1% à 80 % en poids par rapport au poids total de la composition, de préférence allant de 5 % à 60 % en poids, et plus préférentiellement de 5 % à 50 % en poids.

30. Kit selon l'une quelconque des revendications précédentes, **caractérisé en ce que** la première et/ou la seconde composition comprennent une phase aqueuse en une teneur allant de 5% à 80% en poids, par rapport au poids total de la composition, et notamment de 10 % à 70 % en poids.

31. Kit selon la revendication précédente, **caractérisé en ce que** la première et/ou la seconde composition comprend de l'eau en une teneur allant de 10 à 75% en poids par rapport au poids total de la composition, de préférence allant de 15 à 65% en poids.

32. Kit selon l'une quelconque des revendications précédentes, **caractérisé en ce que** la première et/ou seconde composition du kit comprend au moins une matière colorante pulvérulente comprenant au moins un pigment et/ou une nacre.

33. kit selon la revendication précédente, **caractérisé en ce que** le pigment et/ou la nacre est présent dans la première composition du kit en une teneur allant de 0,01 à 6% en poids, par rapport au poids total de la composition, de préférence allant de 0,5 à 5% en poids, et préférentiellement allant de 1 à 3% en poids.

34. kit selon l'une quelconque des revendications 1 à 32, **caractérisé en ce que** la première composition ne contient pas de pigments.

35. kit selon la revendication 32, **caractérisé en ce que** le pigment est présent dans la seconde composition du kit en une teneur allant de 0,01 à 20% en poids, par rapport au poids total de la composition, de préférence allant de 0,5 à 18% en poids, et préférentiellement allant de 3 à 15% en poids.

36. kit selon l'une quelconque des revendications précédentes, **caractérisé en ce que** la première et/ou seconde composition du kit comprend au moins une charge.

37. kit selon l'une quelconque des revendications précédentes, **caractérisé en ce que** la première et/ou la seconde composition du kit sont sous forme d'émulsion eau-dans-huile.

38. kit selon l'une quelconque des revendications précédentes, **caractérisé en ce que** la première et la seconde composition du kit sont sous forme d'émulsion eau-dans-huile.

39. Procédé cosmétique (non thérapeutique) de maquillage de la peau comprenant les étapes suivantes :
i) former un premier dépôt sur lesdites matières kératiniques ;
ii) sur tout ou partie du premier dépôt, former un second dépôt,
lesdits premier et second dépôts résultant de l'application d'une première et seconde compositions du kit selon l'une quelconque des revendications 1 à 38.

40. Utilisation d'un kit de maquillage selon l'une quelconque des revendications 1 à 38, pour obtenir un maquillage uniforme, et/ou masquant les défauts de la peau et/ou évitant le marquage des rides ou ridules.
